(19) Europäisches Patentamt
European Patent Office
Office européen des brevets

(11) Veröffentlichungsnummer: **0 298 336 A1**

(12) **EUROPÄISCHE PATENTANMELDUNG**

(21) Anmeldenummer: **88110254.5**

(51) Int. Cl.⁴: **C07C 119/048 , C09J 3/00**

(22) Anmeldetag: **28.06.88**

(30) Priorität: **08.07.87 DE 3722499**

(43) Veröffentlichungstag der Anmeldung:
**11.01.89 Patentblatt 89/02**

(84) Benannte Vertragsstaaten:
**AT BE CH DE ES FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Sanders, Josef Dr.**
**Wolfskaul 6**
**D-5000 Koeln 80(DE)**
Erfinder: **Hombach, Rudolf Dr.**
**Johann Janssen Strasse 24**
**D-5090 Leverkusen(DE)**
Erfinder: **Reiff, Helmut Dr.**
**Paul Klee Strasse 68 i**
**D-5090 Leverkusen(DE)**
Erfinder: **Dieterich, Dieter Dr.**
**H.T.v. Boettinger Strasse 16**
**D-5090 Leverkusen(DE)**
Erfinder: **Dollhausen, Manfred Dr.**
**Herzogenfeld 21**
**D-5068 Odenthal(DE)**

(54) **Aromatische Polyisocyanate, Verfahren zu ihrer Herstellung sowie ihre Verwendung als Klebstoffkomponente.**

(57) Neue Isocyanatophenoxygruppen aufweisende Verbindungen der allgemeinen Formel

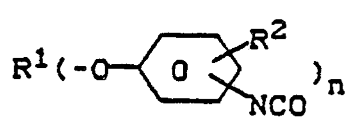

in welcher
$R^1$ für einen n-wertigen Rest steht, wie er durch Entfernung der Hydroxylgruppen von einer n-wertigen Polyhydroxylverbindung des Molgewichtes 92 bis 6000 steht,
$R^2$ für eine Methylgruppe oder Wasserstoff steht und
n eine ganze Zahl von 3 bis 8 bedeutet, sind geeignet für die Herstellung von Klebstoffen.

EP 0 298 336 A1

# Aromatische Polyisocyanate, Verfahren zu ihrer Herstellung sowie ihre Verwendung als Klebstoffkomponente

Die Erfindung betrifft neue, drei oder mehr Isocyanatophenoxygruppen aufweisende Verbindungen, ein Verfahren zu ihrer Herstellung und ihre Verwendung als Härterkomponente bei der Herstellung von Klebstoffen.

Zahlreiche aromatische Polyisocyanate, die mehr als zwei Isocyanatgruppen pro Molekül aufweisen, sind bereits bekannt. Es ist weiterhin bekannt, daß derartige Polyisocyanate als Ausgangsmaterialien zur Herstellung von Klebstoffen potentiell geeignet sind. Representative Vertreter dieser Verbindungsklasse, die technische Bedeutung erlangt haben, werden beispielsweise im Kunststoff Handbuch, Bd. 7: Polyurethane, Hrsg. G. Oertel, 2. Auflage, Hanser Verlag, München 1983, Seiten 15 bis 15 und 581 bis 596, beschrieben. Typische Beispiele derartiger Polyisocyanate sind: Tris-(4-isocyanatophenyl)methan, Polymer-Diisocyanatodiphenylmethan, Thiophosphorsäure-tris-(p-isocyanatophenylester), das Addukt aus Trimethylolpropan und 2,4-Diisocyanatotoluol usw.. Insbesondere Thiophosphorsäure-tris-(p-isocyanatophenyl)-ester zeichnet sich durch gute Adhäsion auch an Gummiqualitäten unterschiedlicher Art bei gleichzeitig weitgehender Farblosigkeit aus und wird daher in großem Umfang zum Kleben empfindlicher, hellfarbiger Werkstoffe eingesetzt.

Obwohl die bekannten Polyisocyanate in ihren klebetechnischen Eigenschaften ein hohes Niveau erreicht haben, sind in Anbetracht gestiegener Anforderungen weitere Verbesserungen insbesondere bei der Wärmefestigkeit sowie bei der Verträglichkeit mit lösungsmittelhaltigen Klebstoffen auf Naturkautschuk- und Polychloroprenbasis wünschenswert.

Aufgabe der Erfindung ist es daher, neue Polyisocyanate mit derart verbesserten Eigenschaften zur Verfügung zu stellen.

Gegenstand der Erfindung sind neue aromatische Polyisocyanate der allgemeinen Formel (I)

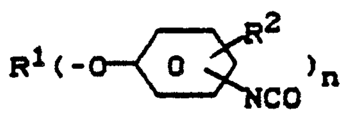

(I)

in welcher $R^1$ für einen n-wertigen Rest steht, wie er durch Entfernung der Hydroxylgruppen von einer n-wertigen Polyhydroxylverbindung des Molgewichtes 92 bis 6000, vorzugsweise 92 bis 400 steht.

$R^2$ für eine Methylgruppe oder vorzugsweise für Wasserstoff steht und

n eine ganze Zahl von 3 bis 8, vorzugsweise 3 bis 6 bedeutet,

Die Verbindungen weisen ausgezeichnete klebetechnische Eigenschaften bei gleichzeitig guten Farbqualitäten auf und sind beispielsweise dem oben erwähnten Thiophosphorsäuretris-(p-isocyanatophenyl)-ester in Bezug auf Wärmefestigkeit der Klebung, Adhäsionsförderung und Verträglichkeit mit lösungsmittelhaltigen Klebstoffen auf Naturkautschuk-und Polychloroprenbasis überlegen.

Gegenstand der Erfindung ist auch ein Verfahren zur Herstellung von drei oder mehr Isocyanatophenoxygruppen aufweisenden Verbindungen der allgemeinen Formel

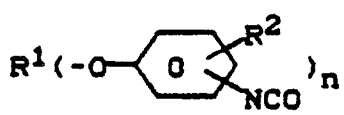

(I)

in der

$R^1$, $R^2$ und n die vorgenannte Bedeutung haben, durch

a) Umsetzung von drei oder mehrwertigen Hydroxylverbindungen der allgemeinen Formel (II)

$R^1(OH)_n$ (II)

mit Verbindungen der allgemeinen Formel (III)

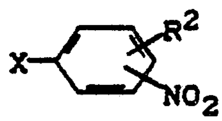

(III)

in der
$R^2$ die vorgenannte Bedeutung hat und
X für Halogen, z.B. Fluor oder vorzugsweise Chlor steht und in der das Halogenatom und die Nitrogruppe vorzugsweise ortho- oder paraständig zueinander angeordnet sind,
in Gegenwart von alkalisch reagierenden Verbindungen zu den entsprechenden Nitrophenoxyaddukten der allgemeinen Formel (IV)

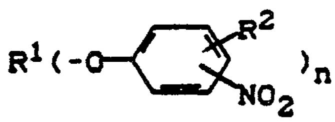

(IV)

in der
$R^1$, $R^2$ und n die vorgenannte Bedeutung haben und

b) an sich bekannte Hydrierung der zuletzt genannten Nitrophenoxyaddukte zu den entsprechenden Aminophenoxyaddukten
und

c) an sich bekannte Umsetzung, insebesondere Phosgenierung der gemäß b) erhaltenen Aminophenoxyaddukte unter Erhalt der erfindungsgemäßen Verbindungen (I).

Gegenstand der Erfindung ist schließlich auch die Verwendung der erfindungsgemäßen Isocyanate insbesondere als Härterkomponente bei der Herstellung von Klebstoffen.

Geeignete mehrwertige Hydroxylverbindungen (II) sind beispielsweise zwei- oder mehrkernige, drei oder mehrwertige Phenole oder vorzugsweise drei- oder mehr Hydroxylgruppen aufweisende araliphatische oder, besonders bevorzugt, aliphatische Alkohole. In Frage kommen beispielsweise Kondensationsprodukte aus Formaldehyd und Phenol, Hydrochinon, Benzcatechin oder Resorcin, ferner Pyrogallol, Phloroglucin, 1,2,4-Trihydroxybenzol, vorzugsweise D(-)-Sorbit, D(-)-Mannit, (±)1,2,6-Hexantriol, Hexantriol (technisches Isomerengemisch), 1,1,1-Trimethylolethan bis 1,1,1-Trimethyloloctadecan, Glycerin, Dipentaerythrit und besonders bevorzugt 1,1,1-Trimethylolpropan und Pentaerythrit. Geeignete alkoholische Ausgangsmaterialien sind auch Anlagerungsprodukte von Epoxiden wie z.B. Ethylenoxid, Propylenoxid, Butylenoxid, Styroloxid, Epichlorhydrin oder höhergliedrige cyclische Ether wie Tetrahydrofuran und Tetrahydropyran an die genannten Polyhydroxylverbindungen, die unter Säurekatalyse, z.B. in Gegenwart von $BF_3$, oder Basenkatalyse nach bekannten Verfahren hergestellt werden können. Dabei können diese Epoxide entweder in reiner Form, gegebenenfalls im Gemisch oder nacheinander an die genannten Polyhydroxylverbindungen addiert werden. Geeignet ist auch der Einsatz von partiell veresterten mehrwertigen Polyhydroxylverbindungen wie z.B. von Pentaerythritmonofettsäureester. Es kann unter Umständen vorteilhaft sein, Mischungen der genannten Polyhydroxylverbindungen einzusetzen.

Bevorzugte Verbindungen (III) sind beispielsweise 2-Nitrochlorbenzol, 2-Nitrofluorbenzol, 4-Nitrochlorbenzol, 4-Nitrofluorbenzol, 1-Methyl-2-nitro-3-chlorbenzol, 1-Methyl-2-nitro-3-fluorbenzol, 1-Methyl-4-nitro-5-chlorbenzol, 1-Methyl-4-nitro-5-fluorbenzol, 1-Methyl-2-nitro-6-chlorbenzol oder 1-Methyl-2-nitro-6-fluorbenzol. Besonders bevorzugte Ausgangsmaterialien (III) sind 2-Nitrochlorbenzol oder 4-Nitrochlorbenzol.

Als alkalisch reagierende Verbindung, die zur Umsetzung der Polyhydroxylverbindungen (II) mit den Halogennitrobenzolen (III) erforderlich sind, kommen beispielsweise Metallhydride, Metallalkoxide und bevorzugt Metallhydroxide in Frage. Besonders bevorzugt werden Natriumhydroxid und Kaliumhydroxid.

Bei der Durchführung der Stufe a) des erfindungsgemäßen Verfahrens können die Ausgangsmaterialien (III) bezogen auf die Komponente (II) sowohl in stöchiometrischer Menge als auch in Über- oder Unterschuß zum Einsatz gelangen. Vorzugsweise wird die Komponente (III) so bemessen, daß auf jedes Mol an Hydroxylgruppen der Komponente (II) 1 bis 1,5 Mol der Komponente (III) vorliegt.

Der bei der Reaktion freiwerdende Halogenwasserstoff wird, wie schon erwähnt, durch Zusatz von Metallhydriden, Metalloxiden und Metallhydroxiden gebunden. Dabei wird ihre Menge zumindest so bemessen, daß sie zur Neutralisation des abgespaltenen Chlorwasserstoffs ausreicht. Besonders bevorzugt werden sie in einer Menge verwendet, daß 1 bis 3 Mol Basenäquivalente pro Mol Hydroxylgruppen zur Verfügung stehen.

Die Stufe a) des erfindungsgemäßen Verfahrens kann in Substanz oder zweckmäßig in einem organischen Lösungsmittel, gegebenenfalls in Gegenwart eines Phasentransferkatalysators durchgeführt werden. Dabei können die Reaktionspartner in homogener Phase oder zweiphasig, gelöst, emulgiert oder suspendiert vorliegen.

Geeignete organische Lösungsmittel sind beispielsweise: Benzol, Toluol, Xylol, Chlorbenzol, Dichlorbenzol, Trichlorbenzol, Diethylether, Diisopropylether, tert.-Butylmethylether, Tetrahydrofuran, Dioxan, Ethylenglykoldimethylether, Essigester, Aceton, Methylethylketon, Acetonitril, Furfurol, Methylenchlorid, Chloroform, Tri chlorethylen, Tetrachlorethylen, Nitromethan, Nitropropan. Bevorzugt werden polare aprotische Lösungsmittel wie z.B. Dimethylformamid, Dimethylacetamid, N-Methylpyrrolidon, Tetramethylharnstoff, N-Methylcaprolactam, Dimethylsulfoxid, Tetramethylensulfon, Hexamethylenphosphorsäuretriamid usw. Ganz besonders bevorzugt werden Dimethylformamid, Dimethylsulfoxid und N-Methylpyrrolidon. Selbstverständlich können auch beliebige Gemische derartiger Lösungsmittel eingesetzt werden.

Die Menge des Lösungsmittels wird hierbei im allgemeinen so bemessen, daß die ausreicht, um die Ausgangsmaterialien (II) und (III) klar zu lösen. In der Praxis bedeutet dies, daß die Lösungsmittel im allgemeinen in einer Menge von 50 bis 1.000, bevorzugt 100 bis 500 Gew.-Teilen Lösungsmittel pro 100 Gew.-Teilen des Gemisches aus den Komponenten (II) und (III) zum Einsatz gelangen.

Es kann in manchen Fällen vorteilhaft sein, die Umsetzung in Gegenwart eines Phasentransferkatalysators durchzuführen. Solche Katalysatoren werden z.B. in E. V. und S. S. Dehmlow, Phase Transfer Catalysis, 2. Auflage, Verlag Chemie 1983, beschrieben. Geeignete Katalysatoren sind quaternäre Ammonium- oder Phosphoniumsalze der Formel

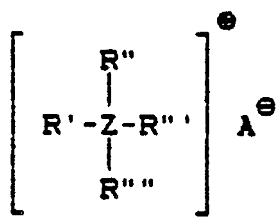

in welcher

Z für Stickstoff oder Phosphor steht,

R', R'', R''' und R'''' für gleiche oder verschiedene Reste stehen und Alkylgruppen mit 1 bis 18 Kohlenstoffatomen bedeuten, wobei einer der Reste auch für einen araliphatischen Rest mit 7 bis 15 Kohlenstoffatomen stehen kann, und wobei die Summe der Kohlenstoffatome der vier Reste vorzugsweise bei 12 bis 31 liegt.

Typische Beispiele geeigneter Katalysatoren sind N-Benzyl-N,N,N-triethyl-ammoniumchlorid oder -bromid, N-Benzyl-N-dodecyl-N,N-dimthyl-ammoniumchlorid oder -bromid, N-Benzyl-N,N,N-tri-n-octyl-ammoniumchlorid oder -bromid oder die diesen Ammoniumsalzen entsprechenden Phosphoniumsalze.

Die beispielhaft genannten quarternären Ammonium- oder Phosphoniumsalze werden bei der Durchführung des erfindungsgemäßen Verfahrens vorzugsweise in Substanz oder in Form ihrer wäßrigen Lösungen (beispielsweise mit einem Feststoffgehalt von 30 bis 60 Gew.-%) und vorzugsweise in einer Menge von 1 bis 10 Mol-%, bezogen auf die Molzahl der vorhandenen Hydroxylgruppen eingesetzt. Bei Verwendung der erfindungsgemäß bevorzugten polaren aprotischen Lösungsmittel wie z.B. Dimethylformamid, N-Methylpyrrolidon oder Dimethylsulfoxid kann ohne Nachteile auf den Zusatz von Phasentransferkatalysatoren verzichtet werden.

Die Stufe a) des erfindungsgemäßen Verfahrens wird im allgemeinen bei 10 bis 100 °C, bevorzugt bei 20 bis 60 °C mit Überdruck, Unterdruck oder zweckmäßig drucklos, kontinuierlich oder diskontinuierlich durchgeführt. Die Verweilzeit beträgt im allgemeinen 0,5 bis 24 Stunden; bevorzugt werden 0,5 bis 8 Stunden. Zur Durchführung der Stufe a) des erfindungsgemäßen Verfahrens kann man beispielsweise so vorgehen, daß man die Ausgangsmaterialien und gegebenenfalls den Phasentransferkatalysator im gewählten Lösungsmittel vorlegt und die Base in gelöster oder suspendierter Form, bevorzugt in fester, möglichst fein gemahlener Form, unter Rühren, gegebenenfalls unter Kühlung portionsweise oder kontinuierlich zugibt. Anschließend rührt man bei Raumtemperatur oder gegebenenfalls erhöhter Temperatur so lange nach, bis IR-spektroskopisch ein vollständiger Umsatz der anfangs vorhandenen Hydroxylgruppen angezeigt wird und/oder dünnschicht- oder gaschromatographisch kein Ausgangsstoff (III) mehr nachgewiesen werden kann. Die Aufarbeitung der Nitrophenoxiverbindungen erfolgt in an sich bekannter Weise. Die Nitrophenoxyverbindungen stellen oftmals in dem verwendeten Lösungsmittel schwer lösliche Substanzen dar, so daß

die Hauptmenge beim Abkühlen des Reaktionsgemisches auf Raumtemperatur ausfällt und durch einfaches Filtrieren isoliert werden kann. Eine andere Art der Aufarbeitung besteht darin, daß Reaktionsgemisch in Wasser einzurühren und das ausgefallene Reaktionsprodukt in üblicher Weise durch Filtration zu gewinnen. Im Falle öliger Reaktionsprodukte ist demgegenüber eine extraktive Aufarbeitung nach üblichen Methoden zweckmäßig, wobei als Extraktionsmittel für das mit Wasser vermischte Reaktionsgemisch beispielsweise Toluol, Methylenchlorid, Chlorbenzol, Dichlorbenzol, 1,2-Dichlorethan, Trichlorethylen u.a. in Betracht kommen.

Es ist auch möglich, das in der Stufe a) anfallende Reaktionsgemisch, gegebenenfalls nach Neutralisation des überschüssigen Alkalihydroxids ohne Zwischenisolierung direkt der Stufe b) zuzuführen.

Die in der Stufe a) des erfindungsgemäßen Verfahrens erhaltenen endständige Nitrophenoxygruppen aufweisenden Verbindungen werden in der Stufe b) in an sich bekannter Weise durch Reduktion mit nascierendem oder katalytischem, beispielsweise mittels Raney-Nickel oder Palladium auf Kohle angeregtem Wasserstoff in die entsprechenden Polyamine überführt. Die Hydrierung kann in Gegenwart oder Abwesenheit von inerten Lösungsmitteln bei 20 bis 120 °C und einem Druck von 20 bis 80 bar erfolgen. Geeignete Lösungsmittel sind beispielsweise Methanol, Ethanol, i-Propanol, Toluol, DMF u.a. Bevorzugt werden DMF und Methanol.

Die resultierenden Polyamine werden als Destillationsrückstand bei der destillativen Entfernung des Lösungsmittels gewonnen und können zumeist, gegebenenfalls nach Waschen mit einem geeigneten organischen Lösungsmittel, ohne weitere Reinigungsschritte weiterverarbeitet werden.

Die in der Stufe b) des erfindungsgemäßen Verfahrens anfallenden Polyamine werden anschließend in der Stufe c) des erfindungsgemäßen Verfahrens mit Phosgen zu den entsprechenden Polyisocyanaten umgesetzt. Hierbei kann man sowohl die freien Amine als auch deren Addukte mit Chlorwasserstoff oder Kohlendioxid einsetzen. Die Phosgenierung wird im allgemeinen in Chlorbenzol oder Dichlorbenzol durchgeführt, einem Reaktionsmedium, welches für die freien Amine in Abhängigkeit von ihrer Konstitution ein Suspendier- oder Lösungsmittel und für die Isocyanate im allgemeinen ein Lösungsmittel darstellt. Die erfindungsgemäße Phosgenierung erfolgt im übrigen nach an sich bekannten Methoden wie sie beispielsweise in der DE-A-3 442 689, in Liebigs Annalen der Chemie, Band 562, Jahrgang 1949, Seiten 75 bis 109, in Ullmanns Encyclopädie der technischen Chemie, Band 14, 4. Auflage, 1977, Seiten 350 bis 354 oder in Houben-Weyl, Methoden der organischen Chemie, Band E 4, 4. Auflage, 1983, Seiten 741 bis 753 beschrieben sind.

Es ist auch möglich, die erfindungsgemäßen Isocyanate nach phosgenfreien Verfahren herzustellen, wie sie beispielsweise in der letztgenannten Literaturstelle S 761 ff beschrieben sind. Beispielsweise lassen sich Amine durch Umsetzung mit Nickelcarbonyl oder Kohlenmonoxid in Gegenwart von Edelmetallkatalysatoren in Isocyanate überführen. Ferner können z.B. auch Nitroverbindungen mit Kohlenmonoxid in Gegenwart von Edelmetallen bzw. Edelmetallkomplexverbindungen zu Isocyanaten umgesetzt werden.

Die nach Aufarbeitung erhaltenen, erfindungsgemäßen drei- oder mehrfunktionellen aromatischen Isocyanate stellen im allgemeinen viskose Öle bzw. niedrig schmelzende Feststoffe dar und zeichnen sich neben ausgezeichneter Adhäsion an Werkstoffen wie Gummi, Metallen, Gläsern usw. durch ihre vergleichsweise geringe Eigenfarbe aus. Sie sind daher, wie bereits oben ausgeführt, besonders zur Herstellung von Klebstoffen geeignet. Dabei werden die erfindungsgemäßen Polyisocyanate anstelle der bislang für diesen Verwendungszweck eingesetzten Polyisocyanate mit den bekannten Reaktionspartnern zur Umsetzung gebracht. Geeignete Ausgangskomponenten für die Herstellung von Klebstoffen werden beispielsweise im Kunststoff Handbuch, Bd. 7: Polyurethane, Hrsg. G. Oertel, 2. Auflage, Hanser Verlag, München 1983, Seiten 581 bis 596 oder auch in Ullmanns Encyclopädie der technischen Chemie, Bd. 14, 4.Auflage, Verlag Chemie, Weinheim 1977, Seiten 227 bis 268 genannt. Dort finden sich auch Hinweise auf die bei der Klebstoff-Herstellung und -Anwendung gegebenenfalls mitzuverwendenden Hilfs- und Zusatzstoffe.

Zur Herstellung von Klebstoffen werden die erfindungsgemäßen Polyisocyanate mit den für diesen Verwendungszweck üblicherweise zur Anwendung kommenden Bindemittel kombiniert. Derartige Bindemittel sind beispielsweise:

1. die üblicherweise als Klebstoff bzw. Klebstoff-Rohstoff eingesetzten Naturkautschuk-Typen;

2. die üblicherweise als Klebstoff bzw. Klebstoff-Rohstoff eingesetzten Synthesekautschuk-Typen; wie z.B. Polymerisate von Dienen wie Butadien oder Mischpolymerisate von Dienen wie Butadien mit einfach olefinisch ungesättigten Verbindungen wie z.B. Styrol, Acrylnitril oder Methacrylnitril, Polymerisate bzw. Mischpolymerisate des 2-Chlor-butadien-1,3 mit anderen olefinisch ungesättigten Monomeren beispielsweise der obengenannten Art, insbesondere eines Chlorgehaltes von ca. 36 Gew.-% oder

3. die üblicherweise als Klebstoff bzw. Klebstoff-Rohstoff eingesetzten Hydroxylgruppen aufweisenden Polyurethane, i.a. des gelchromatographisch ermittelbaren Molekulargewichts von 30 000 bis 1 000 000, vorzugsweise 50 000 bis 200 000, insbesondere lineare oder weitgehend lineare, endständige

Hydroxylgruppen aufweisende Polyurethane die aus (i) Dihydroxypolyestern des rechnerischen Molekulargewichts 1250 bis 4000, vorzugsweise 2000 bis 4000 aus aliphatischen oder aromatischen Dicarbonsäuren wie Adipinsäure oder Phthalsäure und Alkandiolen wie Ethylenglykol, Tetramethylenglykol und/oder Hexamethylenglykol oder aus Polylactonen, insbesondere Poly-ε-caprolacton und (ii) aromatischen oder aliphatischen Diisocyanaten, insbesondere 2,4-Diisocyanatotoluol, dessen Gemischen mit 2,6-Diisocyanatotoluol, 4,4-Diisocyanatodiphenylmethan oder Hexamethylendiisocyanat gegebenenfalls unter Mitverwendung von (iii) Glykolen des Mole kulargewichts 62 bis 200 als Kettenverlängerungsmittel wie z.B. Tetramethylenglykol oder Hexamethylenglykol in einem molaren NCO/OH-Äquivalentverhältnis von 0,9:1 bis 0,999:1 hergestellt werden sind.

Zu den erfindungsgemäß besonders bevorzugten Klebstoff-Klebemitteln gehören die beispielhaft genannten Polymerisate bzw. Mischpolymerisate des 2-Chlorbutadien-1,3 (Polychloropren) und die zuletzt genannten Hydroxylgruppen aufweisenden Polyurethane.

Die genannten Bindemittel bzw. Klebstoffrohstoffe gelangen erfindungsgemäß vorzugsweise als 10- bis 30 gew.-%ige Lösungen in Lösungsmitteln bzw. Lösungsmittelgemischen der bereits beispielhaft genannten Art zum Einsatz.

Die erfindungsgemäß zu verwendenden Polyisocyanatgemische werden diesen Lösungen in Mengen von 2 bis 15 Gew.-%, bezogen auf Bindemittel der genannten Art zugesetzt. Um eine schnelle und homogene Durchmischung der Klebstofflösung mit dem Polyisocyanat zu erreichen, ist es oft vorteilhaft, die erfindungsgemäß zu verwendenden Polyisocyanatgemische als 10 bis 50 gew.-%ige Lösung in derartigen Lösungsmitteln den Bindemittellösungen zuzusetzen.

Zur Modifizierung ihrer klebetechnologischen Eigenschaften können den Klebemitteln auch noch weitere Zusatzmit tel zugemischt werden. Hierzu gehören beispielsweise Naturharze oder modifizierte Harze wie Kolophoniumester oder synthetische Harze wie Phthalharze oder - insbesondere im Fall der Klebstoffe auf Basis von Hydroxylgruppen aufweisenden Polyurethanen auch Polymere wie z.B. Chlorkautschuk oder lösliche Polymerisate oder Mischpolymerisate des Vinylacetats oder andere Vinylverbindungen. Dieses Zusatzmittel, insbesondere die beispielhaft genannten Harze werden im allgemeinen zwecks Erzielung einer besonders andauernden Kontaktbindefähigkeit oder zwecks Erhöhung des Kohäsionsfestigkeit zugesetzt.

Die die Polyisocyanatgemische enthaltenden Klebstofflösungen eignen sich zum Kleben beliebiger Werkstoffe gleicher oder verschiedener Art, z.B. zum Verkleben von Leder, Textilien, Kunststoffen, Holz und Papier, vorzugsweise zum Verkleben von Gummi- oder Weich-PVC.

Darüber hinaus eignen sich die erfindungsgemäßen Polyisocyanate beispielsweise auch zur (Mit-)Verwendung bei der Herstellung von Polyurethanen bzw. Polyurethanharnstoffen, sowie für alle anderen an sich bekannten Reaktionen von Isocyanaten wie z.B. Carbodiimidisierung, Uretdionisierung, Isocyanuratbildung, Amidbildung usw.

Im übrigen kann darauf verwiesen werden, daß die in der Stufe b) des erfindungsgemäßen Verfahrens als Zwischenprodukte anfallenden Polyamine nicht nur als Vorprodukte für die Diisocyanate geeignet sind, sondern ausgezeich nete Vernetzer oder Kettenverlängerungsmittel für Kunststoffvorläufer, beispielsweise für NCO-Prepolymere bei der Herstellung von Polyurethanelastomeren oder für Epoxidharze darstellen, wobei die Polyamine anstelle der bislang für derartige Einsatzgebiete eingesetzten Polyamine verwendet werden.

Die nachfolgenden Beispiele dienen zur weiteren Erläuterung der Erfindung. Alle Prozentangaben beziehen sich auf Gewichtsprozente. Die mittlere Kongröße des eingesetzten gepulverten Natriumhydroxids liegt bei 6 bis 9 µm.

## Beispiele

### Beispiel 1

a) Nitrophenylierung

Zu einer Lösung von 92 g Glycerin (1 Mol) und 519,8 g (3,3 Mol) 4-Chlornitrobenzol in 1 l Dimethylsulfoxid (DMSO) gibt man bei 50 °C unter gutem Rühren über 2 Stunden 180 g (4,5 Mol) gepulvertes Natriumhydroxid in kleinen Portionen zu und rührt anschließend weitere 4 Stunden bei dieser Temperatur. Nach Abkühlen auf Raumtemperatur wird das ausgefallene Produkt abgesaugt, mit Wasser neutral gewaschen, noch einmal mit Essigester gewaschen und anschließend im Vakuum bei 100 °C getrocknet.
Ausbeute: 319 g (70 % der Theorie)
Fp.: 208 bis 209 °C (schwach gelbliches Pulver)

b) Hydrierung

1215 g (2,67 Mol) Nitrophenoxyaddukt gemäß Beispiel 1a) werden in 5 l Methanol in Gegenwart von 125 g Raney-Nickel bei 70 °C und 50 bar hydriert. Nach beendeter Wasserstoffaufnahme wird vom Katalysator abfiltriert und das Lösungsmittel im Vakuum abgezogen.
Ausbeute: 955 g (98 % der Theorie)
Dunkles Öl (GC: 96 %ig)

c) Phosgenierung

In eine Emulsion von 156 g (0,41 Mol) Aminophenoxyaddukt gemäß Beispiel 1b) in 2,2 l Chlorbenzol leitet man unter Rühren bei -10 °C zügig ca. 300 g Phosgen ein. Anschließend wird das Gemisch unter langsamen Einleiten von Phosgen (40 bis 50 g/h) im Verlauf von 5 Stunden bis zum Rückfluß erhitzt und eine weitere Stunde am Rückfluß gekocht, wobei eine fast klare Lösung entsteht. Überschüssiges Phosgen wird durch Ausblasen mit Stickstoff entfernt und das ungelöste Material abfiltriert. Nach Abziehen des Lösungsmittels erhält man ein bräunliches, viskoses Rohprodukt, das durch Behandeln mit neutralem Kieselgel der Korngröße 0,063 bis 0,2 mm weitgehend entfärbt werden kann.
Ausbeute: 151 g (83 % der Theorie) (hellgelbes viskoses Öl)
NCO-Gehalt: Ber.: 28,4 %, Gef.: 27,8 %

Beispiel 2

a) Nitrophenylierung

Zu einer Lösung von 120 g (1 Mol) 1,1,1-Trimethylolethan und 519,8 g (3,3 Mol) 4-Chlornitrobenzol in 1,4 l Dimethylsulfoxid gibt man unter Rühren über 2 Stunden 180 g (4,5 Mol) gepulvertes Natriumhydroxid in kleinen Portionen zu, wobei durch Wasserkühlung die Innentemperatur zwischen 40 bis 50 °C gehalten wird. Nach Zugabe rührt man noch weitere 4 Stunden bei Raumtemperatur und arbeitet analog Beispiel 1a) auf.
Ausbeute: 397 g (82 % der Theorie)
Fp.: 189 bis 191 °C (fast farbloses Pulver)

b) Hydrierung

Analog Beispiel 1b) werden 380 g (0,79 Mol) Nitrophenoxyaddukt gemäß Beispiel 2a) in 1,6 l Methanol in Gegenwart von 40 g Raney-Nickel hydriert.
Ausbeute: 263 g (85 % der Theorie)
Fp.: 137 bis 139 °C (fast farbloses Pulver)

c) Phosgenierung

In eine Suspension von 150 g (0,38 Mol) Aminophenoxyaddukt gemäß Beispiel 2b) in 2,2 l Chlorbenzol leitet man unter Rühren bei -10 °C zügig ca. 300 g Phosgen ein. Die Phosgenierung wird wie in Beispiel 1c) beschrieben zu Ende geführt.
Ausbeute: 156 g (87 % der Theorie) (hellgelbes viskoses Öl)
NCO-Gehalt: Ber.: 26,8 %, Gef.: 26,2 %

## Beispiel 3

a) Nitrophenylierung

134 g (1 Mol) 1,1,1-Trimethylolpropan, 519,8 g (3,3 Mol) 4-Chlornitrobenzol und 180 g (4,5 Mol) gepulvertes Natriumhydroxid werden analog Beispiel 1a) in 1,4 l DMSO umgesetzt.
Ausbeute: 457 g (92 % der Theorie)
Fp.: 172 bis 174 °C (fast farbloses Pulver)

Bei der analogen Umsetzung in 1,5 l Dimethylformamid (DMF) anstelle von DMSO als Lösungsmittel erhält man: Ausbeute: 393 g (79 % der Theorie)

b) Hydrierung

Analog Beispiel 1b) werden 400 g (0,8 Mol) Nitrophenoxyaddukt gemäß Beispiel 3a) in 1,6 l Methanol in Gegenwart von 45 g Raney-Nickel hydriert. Das Rohprodukt wird mit kaltem Toluol gewaschen.
Ausbeute: 306 g (94 % der Theorie)
Fp.: 117 bis 118 °C (hellgraues Pulver)

Phosgenierung

Analog Beispiel 2c) werden 150 g (0,37 Mol) Aminophenoxyaddukt gemäß Beispiel 3b) in 2,2 l Chlorbenzol phosgeniert.
Ausbeute: 160 g (89 % der Theorie) (hellgelbes, viskoses Öl)
NCO-Gehalt: Ber.: 26 %, Gef.: 25,3 %

## Beispiel 4

a) Nitrophenylierung

136 g (1 Mol) Pentaerythrit, 693 g (4,4 Mol) 4-Chlornitrobenzol und 240 g (6 Mol) gepulvertes Natriumhydroxid werden analog Beispiel 2a) in 1,8 l DMSO umgesetzt.
Ausbeute: 595 g (96 % der Theorie)
Fp.: 285 bis 286 °C (hellgelbes Pulver)

Bei der analogen Umsetzung in 2 l N-Methylpyrrolidon anstelle von DMSO als Lösungsmittel erhält man: Ausbeute: 521 g (84 % der Theorie)

b) Hydrierung

Analog Beispiel 1b) werden 400 g (0,65 Mol) Nitrophenoxyaddukt gemäß Beispiel 4a) in 1,8 l DMF in Gegenwart von 60 g Raney-Nickel hydriert. Das Rohprodukt wird mit kaltem Toluol gewaschen.
Ausbeute: 299 g (92 % der Theorie)
Fp.: 210 bis 211 °C (hellgraues Pulver)

c) Phosgenierung

Analog Beispiel 2c) werden 150 g (0,3 Mol) Aminophenoxyaddukt gemäß Beispiel 4b) in 2,2 l Chlorbenzol phosgeniert.
Ausbeute: 174 g (96 % der Theorie) (gelbliches, viskoses Öl)
NCO0-Gehalt: Ber.: 27,8 %, Gef.: 27,3 %

Beispiel 5

a) Nitrophenylierung

136 g (1 Mol) Pentaerythrit, 693 g (4,4 Mol) 2-Chlornitrobenzol und 240 g (6 Mol) gepulvertes Natriumhydroxid werden analog Beispiel 2a) in 2,3 l DMSO umgesetzt.
Ausbeute: 492 g (79 % der Theorie)
Fp.: 214 bis 217 °C (fast farbloses Pulver)

b) Hydrierung

Analog Beispiel 1b) werden 480 g (0,77 Mol) Nitrophenoxyaddukt gemäß Beispiel 5a) in 1,6 l DMF in Gegenwart von 50 g Raney-Nickel hydriert.
Ausbeute: 348 g (90 % der Theorie)
Fp.: 138 bis 139 °C (fast farbloses Pulver)

c) Phosgenierung

Analog Beispiel 2c) werden 150 g (0,3 Mol) Aminophenoxyaddukt gemäß Beispiel 5b) in 2,2 l Chlorbenzol phosgeniert.
Ausbeute: 158 g (87 % der Theorie)
Fp: 177-178 °C (farblose Kristalle)
NCO-Gehalt: Ber.: 27,8 %, Gef.: 27,4 %

Beispiel 6

a) Nitrophenylierung

134 g (1 Mol) (±)-1,2,6-Hexantriol, 519,8 g (3,3 Mol) 4-Chlornitrobenzol und 180 g (4,5 Mol) gepulvertes Natriumhydroxid werden analog Beispiel 2a) in 2,2 l DMSO umgesetzt.
Ausbeute: 352 g (71 % der Theorie)
Fp.: 115 bis 116 °C (gelbliches Pulver)

b) Hydrierung

Analog Beispiel 1b) werden 400 g (0,8 Mol) Nitrophenoxyaddukt gemäß Beispiel 6a) in 1,6 l Methanol in Gegenwart von 50 g Raney-Nickel hydriert.
Ausbeute: 290 g (89 % der Theorie)
Dunkles, viskoses Öl

c) Phosgenierung

Analog Beispiel 1c) werden 150 g (0,37 Mol) Aminophenoxyaddukt gemäß Beispiel 6b) in 2,2 l Chlorbenzol phosgeniert.
Ausbeute: 154 g (86 %) (hellbraunes, viskoses Öl)
NCO-Gehalt: Ber.: 26 %, Gef.: 25,4 %

## Anwendungsbeispiele

### Polyurethan-Klebstoff

Ein im wesentlichen lineares, Hydroxylgruppen-aufweisendes Polyurethan mit einem gelchromatographisch ermittelten Molekulargewicht von etwa 100 000, aus 2,4-Toluylendiisocyanat und einem Hydroxylgruppen-haltigen Polyester aus Adipinsäure und Ethylenglykol wurde in Methylethylketon zu einer etwa 20 %igen Lösung mit einer Viskosität von 2 Pa.s bei 20 °C gelöst. 100 Teile dieser Polyurethan-Lösung wurden mit 5 Teilen der Polyisocyanat-Lösung gründlich gemischt.

### Polyisocyanate

Die Polyisocyanate der Beispiele 3c) und 4c) werden in Ethylacetat zu einer Lösung mit einem Isocyanatgehalt von 5,4 % gelöst. Als Vergleichsbeispiel wurde eine ca. 20 %ige Lösung von Thiophosphorsäure-tris-(p-isocyanato)phenylester in Methylenchlorid mit einem Isocyanatgehalt von 5,4 % verwendet, die z.B. aus DE-PS 1 126 379, Beispiel 2 bekannt ist.

### Testmaterialien

Die Prüfung der Klebstoffe erfolgt nach DIN 53 273 auf den nachstehend beschriebenen Testmaterialien.
Testmaterial A: Styrol-Butadien Gummisohlenmaterial der Shore-A-Härte 90.
Testmaterial B: PVC mit einem Gehalt von 30 % Dioctylphthalat als Weichmacher.

### Klebungen

Zur Prüfung der Klebstoffe wurden aus den Testmaterialien nach DIN 53 273 Prüfkörper hergestellt. Vor dem Aufbringen des Klebstoffes wurde das Gummimaterial mit Schleifpapier der Körnung 40 gründlich gerauht. Der Klebstoffauftrag erfolgte beidseitig zweimal, so daß auf jeder Seite ca. 50 g/m² bezogen auf Feststoff, des Klebstoffs vorlagen.

Nach einer Abdunstzeit von 30 sec wird der Klebfilm einer Seite durch Strahlungswärme (4 sec auf Gerät der Firma Funck AG, München) auf eine Temperatur von ca. 80 °C erwärmt, dann die Klebung zusammengelegt und 10 sec mit 0,4 MPa gepreßt. Nach dem Kleben wurden die Prüfkörper zunächst 9 Tage bei 20 °C gelagert. Der im Trennversuch bei einem Spindelvorschub von 100 mm/min nach DIN 53 273 bei 23 °C ermittelte Schälwiderstand ist in nachfolgender Tabelle aufgeführt:

| Testmaterial | Schälwiderstand N/mm | | |
|---|---|---|---|
| | Erfindung, Beispiel: | | Vergleichsbeispiel |
| | 3c | 4c | |
| A | 9,6 | 10,6 | 7,5 |
| B | 7,1 | 8,3 | 6,8 |

Gegenüber dem Vergleichsbeispiel zeigen die Klebungen mit den erfindungsgemäßen Isocyanaten höhere Festigkeiten.

Wärmefestigkeit von frischen Klebungen:

Die Wärmefestigkeit von frischen Klebungen wurde unmittelbar nach ihrer Herstellung im Schälversuch ermittelt. Hierzu wurden Klebungen auf Testmaterial A sofort nach ihrer Herstellung 1 h bei 50° C gelagert. Der Schälwiderstand wurde sofort an dem noch 50° C warmen Prüfkörper ermittelt.

| Isocyanat | Schälwiderstand N/mm |
|---|---|
| Beispiel 3c | 4,6 |
| Beispiel 4c | 6,8 |
| Vergleichsbeispiel | 7,1 |

Gegenüber dem Vergleichsbeispiel zeigen die Klebungen mit den erfindungsgemäßen Isocyanaten niedrigere Anfangsfestigkeiten. Dies ist für viele Anwendungszwecke von Vorteil, da so eine längere Zeitspanne für gegebenenfalls erforderliche Korrekturen zur Verfügung steht.

Wärmefestigkeiten von gelagerten Klebungen:

Nach einer Ablüftzeit von 30 min. wurden die Klebstoffoberflächen durch Strahlungswärme innerhalb von 4 sec auf eine Temperatur von 80 bis 85° C gebracht. Danach werden die Klebstreifen so zusammengelegt, daß eine überlappte Fläche von 2,5 x 2,5 cm vorliegt. Die Prüfkörper werden 10 sec mit einem Druck von 0,4 MPa gepreßt.

Zur Ermittlung der Wärmefestigkeit nach ASTM 816 D wurden die jeweils 9 Tage bei Raumtemperatur gelagerten Prüfkörper einem Scherversuch unterworfen. Hierbei wird der Prüfkörper mit einer Masse von 11 kg belastet. Nach 20-minütigem Tempern bei 40° C wird durch Erhöhen der Temperatur um 0,25° C pro Minute die Temperatur ermittelt bei der die Klebung versagt. Die erreichten Temperaturen sind in nachstehender Tabelle aufgeführt.

| Isocyanat | Temperatur °C |
|---|---|
| Beispiel 3c | 102 |
| Beispiel 4c | 109 |
| Vergleichsbeispiel | 91 |

Gegenüber dem Vergleichsbeispiel zeigen die Klebungen mit den erfindungsgemäßen Isocyanaten höhre Wärmefestigkeiten.

**Ansprüche**

1. Isocyanatophenoxygruppen aufweisende Verbindungen der allgemeinen Formel

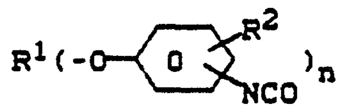

in welcher
$R^1$ für einen n-wertigen Rest steht, wie er durch Entfernung der Hydroxylgruppen von einer n-wertigen Polyhydroxylverbindung des Molgewichtes 92 bis 6000 steht,
$R^2$ für eine Methylgruppe oder Wasserstoff steht und
n eine ganze Zahl von 3 bis 8 bedeutet.

2. Aromatische Polyisocyanate nach Anspruch 1, dadurch gekennzeichnet, daß
$R^1$ für einen n-wertigen Rest steht, wie er durch Entfernung der Hydroxylgruppe von einer n-wertigen Polyhydroxylverbindung des Molgewichts 92 bis 400 steht und in der
n für eine ganze Zahl von 3 bis 6 steht.

3. Aromatische Polyisocyanate nach Anspruch 1 und 2, dadurch gekennzeichnet, daß
$R^1$ für einen aliphatischen Kohlenwasserstoffrest steht.

4. Aromatische Polyisocyanate nach Anspruch 1 bis 3, dadurch gekennzeichnet, daß
$R^2$ für Wasserstoff steht und der Rest $R^1O$- und die Isocyanatgruppe ortho- oder paraständig zueinander angeordnet sind.

5. Verfahren zur Herstellung von aromatischen Polyisocyanaten nach Anspruch 1 bis 4 durch Umsetzung von

a) n-wertigen Hydroxylverbindungen der allgemeinen Formel (II)

$R^1(OH)_n$ (II)

mit Verbindungen der allgemeinen Formel (III)

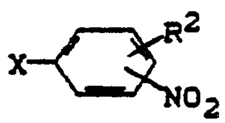 (III)

in Gegenwart von alkalisch reagierenden Verbindungen zu den entsprechenden Nitrophenoxyaddukten der allgemeinen Formel

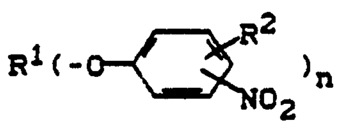 (IV)

wobei in diesen Formeln $R^1$, $R^2$ und n die in den Ansprüchen 1 bis 4 angegebene Bedeutung haben und X für Halogen steht,

b) Hydrierung der zuletzt genannten Nitrophenoxyaddukte zu den entsprechenden Aminophenoxyaddukten

c) an sich bekannte Umsetzung, insbesondere Phosgenierung der gemäß b) erhaltenen Aminophenoxyaddukte unter Erhalt der Verbindungen I.

6. Verfahren nach Anspruch 5, dadurch gekennzeichnet, daß man die Stufe a) des Verfahrens in Gegenwart von gepulvertem Natrium- und/oder Kaliumhydroxid in einer zur Neutralisation des abgespaltenen Halogenwasserstoffs mindestens ausreichenden Menge und gegebenenfalls in Gegenwart eines stark polaren, aprotischen Lösungsmittels durchführt.

7. Verfahren nach Anspruch 5 und 6, dadurch gekennzeichnet, daß man die Stufe a) des Verfahrens in Dimethylformamid, Dimethylsulfoxid oder N-Methylpyrrolidon durchführt.

8. Verfahren nach Anspruch 5 bis 7, dadurch gekennzeichnet, daß X für Chlor steht.

9. Verwendung der aromatischen Polyisocyanate nach Anspruch 1 bis 4 bei der Herstellung von Klebstoffen.

Europäisches Patentamt

# EUROPÄISCHER RECHERCHENBERICHT

Nummer der Anmeldung

EP 88 11 0254

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 183 115 (BAYER)<br>* Ansprüche *<br>--- | 1-9 | C 07 C 119/048<br>C 09 J 3/00 |
| A | DE-A-1 959 462 (FARBENFABRIKEN BAYER)<br>* Ansprüche; Beispiel 1 *<br>----- | 1-9 | |

| RECHERCHIERTE SACHGEBIETE (Int. Cl.4) |
|---|
| C 07 C 119/00 |

Der vorliegende Recherchenbericht wurde für alle Patentansprüche erstellt

| Recherchenort | Abschlußdatum der Recherche | Prüfer |
|---|---|---|
| DEN HAAG | 17-10-1988 | WRIGHT M.W. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)